(19) Europäisches Patentamt
European Patent Office
Office européen des brevets

(11) **EP 3 300 798 A1**

(12) **EUROPÄISCHE PATENTANMELDUNG**

(43) Veröffentlichungstag:
**04.04.2018  Patentblatt 2018/14**

(21) Anmeldenummer: **16191751.3**

(22) Anmeldetag: **30.09.2016**

(51) Int Cl.:
*B01J 23/755* (2006.01)     *B01J 25/02* (2006.01)
*B01J 35/00* (2006.01)     *B01J 35/02* (2006.01)
*B01J 35/04* (2006.01)     *B01J 35/10* (2006.01)
*B01J 19/00* (2006.01)     *B01J 23/16* (2006.01)
*B01J 23/70* (2006.01)     *C07C 31/02* (2006.01)
*C07C 1/20* (2006.01)

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**MA MD**

(71) Anmelder: **Evonik Degussa GmbH**
**45128 Essen (DE)**

(72) Erfinder:
- **WIELAND, Stefan**
  **63456 Hanau (DE)**
- **ROOS, Meike**
  **63654 Büdingen (DE)**
- **POSS, René**
  **63450 Hanau (DE)**

(54) **KATALYSATORFESTBETT ENTHALTEND METALLSCHAUMKÖRPER**

(57)     Die vorliegende Erfindung betrifft ein Festbett aus katalytisch aktiven Metallschaumkörpern mit einem Volumen von nicht mehr als 500 mL, die zu mindestens 95 Gew.-% aus Metallen bestehen.
   Das Festbett kommt für katalytischen Umsetzungen in einem Dreiphasen-Reaktionsgemisch zum Einsatz.

EP 3 300 798 A1

**Beschreibung**

Die vorliegende Erfindung betrifft ein Katalysatorfestbett aus katalytisch aktiven Metallschaumkörpern und ein Verfahren zur katalytischen Umsetzung eines Gemisches aus gasförmigen und flüssigen Edukten in einem Reaktor, in dem dieses Katalysatorfestbett angeordnet ist.

Heterogen katalysierte Verfahren sind in der industriellen Praxis sehr verbreitet. Beispiele für solche Prozesse umfassen Ammoniak-, Methanol-, Fischer-Tropsch-Synthesen, zahlreiche Oxidations- und Hydrierungsprozesse und viele andere Umsetzungen. Einige solcher Verfahren sind ausführlich in Ullmann's Encyclopedia of Industrial Chemistry, Kapitel "Heterogeneous Catalysis and Solid Catalysts, 3. Industrial Applications", veröffentlicht online am 15.10.2011, DOI: 10.1002/14356007.o05_o03, beschrieben.

Viele derartige Prozesse werden in unterschiedlich aufgebauten und auf unterschiedliche Weise betriebenen Festbettreaktoren durchgeführt, siehe z.B. in Ullmann's Encyclopedia of Industrial Chemistry, Kapitel "Catalytic Fixed-Bed Reactors", veröffentlicht online am 15.07.2012, DOI: 10.1002/14356007.o05_o03.

Die Entwicklung neuer, für spezielle Anwendungsgebiete maßgeschneiderter heterogener Katalysatoren stellt eine wichtige Aufgabe moderner industrieller und akademischer Forschung dar. So können solche heterogenen Katalysatoren verschiedene Formen, Partikelgrößen und andere Materialeigenschaften aufweisen, wie z.B. in Ullmann's Encyclopedia of Industrial Chemistry, Kapitel "Heterogeneous Catalysis and Solid Catalysts, 2. Development and Types of Solid Catalysts", veröffentlicht online am 15.10.2011, DOI: 10.1002/14356007.o05_o02, näher beschrieben.

Metallschäume sind in der Fachwelt gut bekannt und werden meistens als funktionelle oder Struktur gebende Materialien verwendet, siehe auch Ullmann's Encyclopedia of Industrial Chemistry, Kapitel "Metallic Foams", veröffentlicht online am 15.07.2012, DOI: 10.1002/14356007.c16_c01.pub2. So finden Nickelschäume beispielsweise Anwendung als Elektroden in Batterien oder als Filtrationselemente.

Katalytische Anwendungen von auf Metallschäumen basierenden Materialien sind sehr beschränkt. Bislang wurden Metallschaumkörper meist für heterogen katalysierte Gasphasenreaktionen eingesetzt. Zu diesen Anwendungen zählen insbesondere die katalytische Nachbehandlung der Abgase von Verbrennungsmotoren, die katalytische Reinigung von Rauchgasen, die Wassergas-Shift-Reaktion zur Herstellung von Wasserstoff aus Kohlenstoffmonoxid und Wasserdampf oder die Dampfreformierung von Methan. In solchen Anwendungen ist der Staudruck, den die Metallschaumkörper aufgrund ihrer hohen Porosität in dem durchströmenden Reaktionsmedium aufbauen, vergleichsweise gering.

Da die im Stand der Technik bekannten porösen Metallschaumkörper selbst nicht katalytisch aktiv sind und über eine für heterogenkatalytischen Anwendungen unzureichende geometrische Oberfläche verfügen, muß für die bekannten Applikationen eine zusätzliche Beschichtung zur katalytischen Aktivierung auf den Metallschaumkörper aufgebracht werden. Dadurch steigt der Gegendruck gegenüber dem durchströmenden Reaktionsmedium signifikant an, was meist erhebliche Effizienzverluste in heterogenkatalytischen Anwendungen zur Folge hat.

DE 10245510 A1 offenbart ein Abgasfilter für Verbrennungsmotoren umfassend einen als Tiefenfilterelement eingesetzten Schaumkörper, der in der Matrix des Werkstoffs verteilte Oxidationskatalysatoren enthält und für die Rußpartikelabtrennung und deren katalytische Nachverbrennung verwendet werden kann.

AICHE Journal, 2015, Vol. 61, No 12, S. 4323-4331 offenbart die Verwendung eines Ni-$Al_2O_3$/Ni-Metallschaumkatalysators als Chips mit 16 mm Durchmesser in der Gasphasenhydrierung von $CO_2$ zu Methan in einem Festbettreaktor. Nickelschaum wird dabei als ein inerter Katalysatorträger verwendet, auf dessen Oberfläche eine aktive Ni/$Al_2O_3$-Beschichtung aufgetragen wird. Der so hergestellte Katalysator enthält 12,6 Gew.-% NiO und 4,2 Gew.-% $Al_2O_3$.

In Catalysis Today, 2016, Vol. 273, S. 221-233 ist ferner beschrieben, dass Metallschäume allgemein keine ausreichend große Oberfläche für katalytische Anwendungen aufweisen. Notwendig ist deswegen die Auftragung eines "Washcoats" mit hoher Oberfläche und die nachfolgende Imprägnierung mit einer katalytisch aktiven Komponente.

Bei großtechnisch-chemischen Anwendungen handelt es sich häufig um Umsetzungen flüssiger, meist organischer Verbindungen mit gasförmigen Reaktanden. Beschichtete katalytische Metallschaumkörper eignen sich für solche Anwendungen nicht, da die meist höher viskosen flüssigen Edukte nicht in die durch die Beschichtung verengten Poren der Metallschaumkörper eindringen können und infolge dessen mit den katalytisch aktiven Zentren in den Poren nicht in Kontakt kommen. In solchen Anwendungen kommen heute meist tablettierte oder extrudierte oxidische Formkörper zum Einsatz, die beispielsweise durch Imprägnierung mit Edelmetalllösungen zuvor katalytisch aktiviert wurden. Diese Formkörper weisen zum Teil zwar hohe BET-Oberflächen auf. Die zugängliche geometrische Oberfläche dieser oxidischen Formkörper ist jedoch begrenzt, so dass die katalytische Effizienz solcher Formkörper in Reaktoren, die typischerweise mehrere Kubikmeter Rauminhalt aufweisen, durch einen schlechten Stofftransport der Reaktanden an das katalytisch aktive Zentrum beschränkt ist.

ACS Catal. 2016, Vol 6, S 5432-5440 offenbart mit Kohlenstoffnanofasern (Carbon Nano Fibres CNFs) beschichtete Nickelschäume und deren Anwendung in der Hydrierung von Nitriten in einem Festbettreaktor in der flüssigen Phase. Die darin beschriebenen Katalysatoren weisen einen relativ hohen Anteil an Kohlenstoff (27 Gew.-%) auf.

Der vorliegenden Erfindung lag die Aufgabe zugrunde, eine effiziente katalytische Umsetzung in einem Dreiphasensystem an einem Katalysatorfestbett zu ermöglichen und dabei sowohl makroskopische Stofftransportlimitierungen als auch Limitierungen durch hohe Staudrücke über dem Katalysatorfestbett, wie sie in Verfahren nach dem Stand der Technik auftreten, zu verringern bzw. zu überwinden, und dazu den entsprechenden Katalysator bereit zu stellen.

Diese Aufgabe wird gelöst durch ein Festbett enthaltend katalytisch aktive Metallschaumkörper zur katalytischen Umsetzung von mindestens einer Eduktkomponente in flüssiger Phase und mindestens einer gasförmigen Komponente, wobei die katalytisch aktiven Metallschaumkörper ein Volumen von nicht mehr als 500 Millilitern aufweisen und zu mindestens 95 Gew.-% aus Metallen bestehen.

Unter Metallschäumen werden feste metallische Schäume verstanden, die eine hohe Porosität und zahlreiche gegenseitige Verbindungen zwischen Bereichen festen Materials mit irregulärer Struktur aufweisen. Solche Metallschäume werden auch "zellulare Metalle" genannt, wobei der Begriff "Metallschäume" verbreiteter ist. Der Begriff "Metallschäume" ist in der Fachliteratur gut etabliert und zum Beispiel in Ullmann's Encyclopedia of Industrial Chemistry, Kapitel "Metallic Foams", veröffentlicht online am 15.07.2012, DOI: 10.1002/14356007.c16_c01.pub2 näher erläutert.

Unter "katalytisch aktiven Metallschaumkörpern" im Sinne dieser Erfindung werden Metallschaumkörper verstanden, die keine zusätzlichen Beschichtungen aufweisen, welche gegebenenfalls eine Verengung der Poren in den Metallschaumkörpern bewirken können. Katalytisch aktive Metallschaumkörper im Sinne dieser Erfindung sind ohne zusätzliche, gegebenenfalls katalytisch aktive Beschichtungen in der jeweiligen Zielreaktion katalytisch aktiv. Auch ein Festbett gemäß der vorliegenden Erfindung enthält keine zusätzlichen, katalytisch aktiven Beschichtungen.

Die erfindungsgemäßen katalytisch aktiven Metallschaumkörper sind äußerlich diskret abgegrenzte, regelmäßig geformte geometrische Körper, deren Volumen sich aus ihren Abmessungen exakt berechnen lässt. Bevorzugt liegen die erfindungsgemäßen katalytisch aktiven Metallschaumkörper als zylindrische, ringförmige, quaderförmige, parallelipedförmige oder würfelförmige Körper vor. Das Volumen eines solchen Körpers ergibt sich allgemein durch Multiplikation der messbaren Grundfläche des Körpers mit seiner ebenfalls messbaren Höhe.

Katalytisch aktive Metallschaumkörper der vorliegenden Erfindung werden als Schüttgut in einem Festbett angeordnet. Unter einem Festbett im Sinne der vorliegenden Erfindung wird ein räumlich fest angeordnetes Gemenge verstanden, d.h. die Schüttung eines oder mehrerer Stoffe bestehend aus einzelnen Körpern, Teilchen oder Partikeln, die in einem Reaktor und/oder in einer Reaktionszone räumlich fixiert vorliegen.

Das erfindungsgemäße Festbett kann neben den katalytisch aktiven Metallschaumkörpern auch andere, gegebenenfalls katalytisch inaktive Bestandteile enthalten, beispielsweise Füllstoffe oder strömungsbrechende und/oder Turbulenz erzeugende Elemente.

Das erfindungsgemäße Festbett besteht bevorzugt zu mehr als 50 Gew.-%, besonders bevorzugt zu mehr als 80 Gew.-%, ganz besonders bevorzugt zu mehr als 95 Gew.-% aus katalytisch aktiven Metallschaumkörpern. Ganz besonders bevorzugt besteht das Festbett der vorliegenden Erfindung aus katalytisch aktiven Metallschaumkörpern.

Die im erfindungsgemäßen Festbett enthaltenen katalytisch aktiven Metallschaumkörper bestehen zu mindestens 95 Gew.-%, bevorzugt zu mindestens 97 Gew.-%, besonders bevorzugt zu mindestens 98 Gew.-%, ganz besonders bevorzugt zu mindestens 99 Gew.-%, aus Metallen.

Unter Metallen werden die Elemente der Gruppe IA (ausschließlich Wasserstoff), IIA, IB-VIIIB (Übergangsmetalle), IIIA (ausschließlich Bor), IVA (hier: Sn und Pb), VA (hier: Bi) und VIA (hier: Po) des Periodensystems der chemischen Elemente verstanden.

Die im erfindungsgemäßen Festbett enthaltenen katalytisch aktiven Metallschaumkörper enthalten bevorzugt ein oder mehrere Metalle ausgewählt aus der Gruppe bestehend aus Nickel, Cobalt, Eisen, Silber, Platin, Chrom, Molybdän und Wolfram. Besonders bevorzugt enthalten die im erfindungsgemäßen Festbett vorliegenden katalytisch aktiven Metallschaumkörper ein oder mehrere Metalle ausgewählt aus der Gruppe bestehend aus Nickel, Cobalt und Eisen. Ganz besonders bevorzugt enthalten die im erfindungsgemäßen Festbett vorliegenden katalytisch aktiven Metallschaumkörper 65 bis 98 Gew.-%, bevorzugt 70 bis 95 Gew.-%, besonders bevorzugt 80 bis 90 Gew. % Nickel oder Cobalt.

Zusätzlich enthalten die im erfindungsgemäßen Festbett vorliegenden katalytisch aktiven Metallschaumkörper bevorzugt bis zu 25 Gew.-%, besonders bevorzugt 2 bis 20 Gew.-%, ganz besonders bevorzugt 4 bis 15 Gew.-% Aluminium. Für die meisten Anwendungen eigen sich besonders gut Ausführungsformen mit 7 bis 13 Gew.-% Aluminium.

Weiterhin enthalten die im erfindungsgemäßen Festbett vorliegenden katalytisch aktiven Metallschaumkörper bis zu 10 Gew.-%, bevorzugt 0,05 bis 5 Gew.-%, besonders bevorzugt 0,1 bis 2 Gew.-% Molybdän (Mo) und/oder 0 bis 10 Gew.-%, bevorzugt 0,05 bis 5 Gew.-%, besonders bevorzugt 1,5 bis 3,5 Gew.-% eines oder mehrerer Elemente ausgewählt aus der Gruppe bestehend aus Eisen und Chrom.

Die im erfindungsgemäßen Festbett enthaltenen katalytisch aktiven Metallschaumkörper weisen darüber hinaus weniger als 5 Gew.-% Sauerstoff in metalloxidischen Verbindungen auf, da das Vorliegen solche oxidischer Strukturen die katalytische Aktivität der erfindungsgemäßen katalytisch aktiven Metallschaumkörper in den entsprechenden Zielreaktionen deutlich minimieren kann. Bevorzugt enthalten die erfindungsgemäßen katalytisch aktiven Metallschaumkörper weniger als 3 Gew.-%, ganz besonders bevorzugt weniger als 1 Gew.-% Oxide. Der Sauerstoffgehalt besonders gut geeigneter katalytisch aktiver Metallschaumkörper liegt bevorzugt unterhalb von 7500 ppm, besonders bevorzugt unter 5000 ppm und ganz besonders bevorzugt unter 3000 ppm auf.

Metall- und Sauerstoffgehalt der erfindungsgemäßen katalytisch aktiven Metallschaumkörper können mit im Stand der Technik bekannten und dem Fachmann geläufigen Methoden der Elementanalyse bestimmt werden, Metallgehalte beispielsweise - nach Durchführung eines geeigneten nasschemischen Aufschlussverfahrens - mittels optischer Emissionsspektroskopie (ICP-OES). Für die Bestimmung der Sauerstoffgehalte eignet sich die Infrarotspektroskopie an resultierendem $CO_2$ beispielsweise mit dem Analysator LECO TCH 600. Vor der Bestimmung des Sauerstoffgehalts der erfindungsgemäßen katalytisch aktiven Metallschaumkörper müssen diese gegebenenfalls gezielt unter Ausschluss von Sauerstoffkontaminationen aus umgebendem Medium deaktiviert werden. Dies gilt insbesondere für Ausführungsformen, die aufgrund ihrer katalytischen Aktivität an Luft pyrophor sein können und deshalb vor Einsatz unter Wasser als Schutzmedium gehandhabt werden. Aus einem solchen Material ist Wasser zunächst schonend zu entfernen, beispielsweise durch vorsichtiges Waschen mit einem trockenen organischen Lösungsmittel wie Ethanol, das eine gewisse Aufnahmefähigkeit für das Restwasser in den Poren der katalytisch aktiven Schaumkörper aufweist. Durch anschließendes Evakuieren werden Lösungsmittelreste und Restwasser entfernt. Die Deaktivierung kann dann durch langsames Aufheizen auf bis zu 200°C im Vakuum oder im Schutzgasstrom (Stickstoff oder Argon) erfolgen.

Im erfindungsgemäßen Festbett liegen die katalytisch aktiven Metallschaumkörper als Schüttgut vor, wobei ein Volumen von 1 L dieses Schüttgutes bevorzugt nicht mehr als 0,8 kg Gewicht aufweist. Besonders bevorzugt weist ein Volumen von 1 L dieses Schüttguts 0,1 bis 0,7 kg, ganz besonders bevorzugt 0,2 bis 0,6 kg Gewicht auf. Dementsprechend weisen die im erfindungsgemäßen Festbett enthaltenen katalytisch aktiven Metallschaumkörper bevorzugt eine Schüttdichte von nicht mehr als 0,8 kg/L, besonders bevorzugt von 0,1 bis 0,7 kg/L, ganz besonders bevorzugt von 0,2 bis 0,6 kg/L auf.

Schüttdichte $d_{Sch}$, manchmal auch Fülldichte (Engl. bulk density, poured density) eines Feststoffes genannt, ist das Verhältnis der Masse zum Volumen eines Gemenges aus einem gegebenenfalls körnigen Feststoff und Luft, die die Hohlräume zwischen den Partikeln des Feststoffs beziehungsweise den Feststoffkörpern ausfüllt. Dieser unter Fachleuten gängige Parameter kann mittels eines Messzylinders und einer Waage durch Bestimmung der Masse ($M_F$) eines definierten Schüttungsvolumens an Feststoff ($V_F$) bestimmt werden:

$$d_{Sch} = M_F/V_F$$

Die im erfindungsgemäßen Festbett enthaltenen katalytisch aktiven Metallschaumkörper weisen ein Volumen von nicht mehr als 500 Millilitern (mL) auf. Geometrische Form und Größe der erfindungsgemäßen katalytisch aktiven Metallschaumkörper und somit deren Volumen ist steuerbar und kann auf die im Einsatzreaktor herrschenden Gegebenheiten angepasst werden, um optimale Bedingungen für den Stofftransport bei minimalem Gegendruck auf das Reaktionsmedium zu erhalten. In bevorzugten Ausführungsformen des erfindungsgemäßen Festbetts weisen die katalytisch aktiven Metallschaumkörper ein Volumen von nicht mehr als 200 mL, besonders bevorzugt von 0,02 bis 100 mL und ganz besonders bevorzugt von 0,02 bis 50 mL auf.

Bevorzugt liegen die katalytisch aktiven Metallschaumkörper im erfindungsgemäßen Festbett als zylindrische, ringförmige, quader-, paralleliped- oder würfelförmige Körper vor. Diese werden bevorzugt aus Metallschaumplatten mit einer Kantenlänge von mindestens 300 mm und einer Dicke von 0,5 bis 10 mm, bevorzugt von 1 bis 5 mm hergestellt. Aus diesen Metallschaumplatten können beispielsweise mittels Laserschneiden beziehungsweise Laserstrahlschneiden kleinere geometrische Körper erhalten werden, die bevorzugt eine Quader- oder Parallelipedform mit einer maximalen Kantenlänge von nicht mehr als 50 mm, bevorzugt von nicht mehr als 25 mm und besonders bevorzugt von nicht mehr als 10 mm aufweisen. In der gleichen Weise können würfelförmige Körper mit einer Kantenlänge von 0,5 bis 10 mm, bevorzugt von 1 bis 5 mm hergestellt werden. Auch ringförmige Körper können mit dieser Herstellungsweise erzeugt werden. Deren Außendurchmesser beträgt vorzugsweise weniger als 100 mm, besonders bevorzugt weniger als 50 mm und ganz besonders bevorzugt weniger als 20 mm. Der Innendurchmesser ist so zu wählen, dass eine Ringbreite von bevorzugt 2 bis 80 mm entsteht.

Die katalytisch aktiven Metallschaumkörper im erfindungsgemäßen Festbett können eine zylindrische Form aufweisen, wobei die zylindrische Form durch Wicklung einer Metallschaumplatte erzeugt worden ist. Insbesondere in großtechnischen Festbettreaktoren mit Gesamtvolumina von mehreren Kubikmetern eignet sich ein erfindungsgemäßes Festbett aus zylindrisch geformten katalytisch aktiven Metallschaumkörper mit einem Volumen von 25 bis 500 Millilitern. Zylindrische Metallschaumkörper können ebenfalls aus Metallschaumplatten mit einer Kantenlänge von mindestens 300 mm und einer Dicke von 0,5 bis 10 mm, bevorzugt von 1 bis 5 mm erzeugt werden. Hierzu werden die Metallschaumplatten über die lange Kante aufgewickelt bis eine zylindrische "Rolle" mit dem gewünschten Zieldurchmesser entstanden ist, und im Anschluss beispielsweise mittels Laserschneiden bzw. Laserstrahlschneiden auf die gewünschte Länge gebracht. Zur Verbesserung des Stoffstransports und Erhöhung der Turbulenz des den katalytisch aktiven Metallschaumkörper in der Anwendung durchströmenden Reaktionsmediums kann die Metallschaumplatte vor Wicklung in eine gewellte Form - ähnlich einem Wellblech - gebracht werden, so dass im resultierenden zylindrischen Metallschaumkörper laminare Strömungswege minimiert werden.

Die im erfindungsgemäßen Festbett enthaltenen katalytisch aktiven Metallschaumkörper weisen eine makroskopische Schaumstruktur auf. Poröse Metallschaumstrukturen, die viele Hohlräume enthalten, können zum Beispiel durch Einwirkung von Gasen auf ein verflüssigtes Metall und nachfolgende Abkühlung entstehen. Eine weitere Möglichkeit, um zu solchen Strukturen zu gelangen, besteht darin, organische Schaumstrukturen als Templat (Basis) für die Auftragung eines Metalls zu benutzen und anschließend das organische Templat durch Verbrennung zu entfernen. Die in den erfindungsgemäßen Metallschaumkörpern vorliegenden makroskopischen Poren haben bevorzugt eine Größe im Bereich von 100 bis 5000 $\mu$m, besonders bevorzugt von 200 bis 2500 $\mu$m, besonders bevorzugt von 400 bis 1200 $\mu$m. Zur Bestimmung der Größe der makroskopischen Poren kann zum Beispiel eine in "The Guide 2000 of Technical Foams", Buch 4, Teil 4, Seiten 33-41 beschriebene Methode benutzt werden. Dabei kann die Größe der makroskopischen Poren durch eine optische Messung des Porendurchmessers einer ausgewählten Pore bestimmt werden. Diese Messung wird für mindestens 100 unterschiedliche Poren wiederholt und daraus als Analysenergebnis ein Mittelwert Porendurchmessers berechnet.

Die im erfindungsgemäßen Festbett enthaltenen katalytisch aktiven Metallschaumkörper weisen bevorzugt eine BET-Oberfläche von 1 bis 200 m$^2$/g, besonders bevorzugt 10 bis 120 m$^2$/g, ganz besonders bevorzugt 70 bis 100 m$^2$/g auf. Die spezifische Oberfläche, auch vereinfacht BET-Oberfläche genannt, wird nach DIN 9277 durch Stickstoffadsorption nach dem Brunauer-Emmett-Teller-Verfahren bestimmt, wie beschrieben in J. Am. Chem. Soc. 1938, Vol. 60, S. 309-319.

Die im erfindungsgemäßen Festbett enthaltenen katalytisch aktiven Metallschaumkörper werden beispielsweise durch chemisches Herauslösen von mindestens einem Metall aus einer mindestens binären Metalllegierung erhalten.

Insbesondere eignen sich aktivierte Nickel- oder Cobaltkatalysatoren vom "Raney-Typ", auch "Raney-Katalysatoren" genannt, für den Einsatz als katalytisch aktive Metallschaumkörper in einem erfindungsgemäßen Festbett. Daher wird die bevorzugte Herstellung eines solchen katalytisch aktiven Metallschaumkörpers nachstehend am Beispiel eines katalytisch aktivierten Nickelschaumkörpers beschrieben. Die erläuterten Herstellungsschritte können bei geeigneter Anpassung der Prozessparameter auf andere, hier nicht explizit beschriebene Metallschaumkörper angewandt werden.

Zur Herstellung eines katalytisch aktiven Nickelschaumkörpers wird ein kommerziell erhältlicher Nickelschaum mit einem Haftvermittler behandelt und mit Aluminiumpulver beschichtet. Jeder Haftvermittler, der die Haftung zwischen Metallen und organischen Materialen verbessert, kann eingesetzt werden. Geeignet ist beispielsweise Polyethyleniminlösung.

In diesen Prozessschritten behält der Nickelschaum seine Duktilität bei und kann beispielsweise für die Herstellung zylindrischer Körper geeignet verformt werden. In einer anschließenden Wärmebehandlung unter Ausschluss von Sauerstoff werden zunächst Feuchtigkeit und organischchemische Rückstände des Haftvermittlers gezielt entfernt. Dann wird Aluminium im Nickelschaum unter Ausbildung intermetallischer Phasen gelöst. Die räumliche Struktur des resultierenden Nickel/Aluminium-Legierungsschaums inklusive der Porenstruktur (Geometrie, Porosität, Dimensionen) entspricht der des eingesetzten Nickelschaums, dessen ursprüngliche Ausprägung in diesem Prozessschritt vollumfänglich erhalten bleibt. Somit enthält der resultierende Nickel/Aluminium-Legierungsschaum keine die Poren verengende Beschichtung.

Die Wärmebehandlung wird vorzugsweise in einem Temperaturbereich zwischen 500 und 1000 °C, besonders bevorzugt zwischen 600 und 750°C durchgeführt. Die Wärmebehandlung erfolgt in einer Atmosphäre aus Sauerstoff-freiem Inertgas, um die Ausbildung störender oxidischer Phasen und Schichten zu verhindern.

Nach der Wärmebehandlung kann eine Zerkleinerung und/oder Vereinzelung des Materials beispielsweise durch Laserschneiden beziehungsweise Laserstrahlschneiden erfolgen, sofern dies nicht bereits in einem Formgebungsschritt vor der Wärmebehandlung geschehen ist.

Aus den derart erhaltenen Nickel/Aluminium-Formkörpern werden erfindungsgemäße katalytisch aktive Nickelschaumkörper durch Herauslösen mindestens eines Teils des darin enthaltenen Aluminiums erzeugt. Hierzu werden wässrige basische Lösungen eingesetzt, bevorzugt Alkalihydroxidlösungen, wobei das Alkalihydroxid ausgewählt ist aus der Gruppe bestehend aus Natriumhydroxid, Kaliumhydroxid oder Lithiumhydroxid. Besonders bevorzugt ist wässrige Natriumhydroxidlösung. Die Konzentration der in diesem Prozessschritt eingesetzten wässrigen Alkalihydroxidlösung liegt allgemein zwischen 0,1 und 60 Gew.-%. Bevorzugt erfolgt das Herauslösen des Aluminiums (auch als Laugung bezeichnet) mit einer 5 bis 50 Gew.-%igen, besonders bevorzugt 5 bis 35 Gew.-%igen wässrigen Natriumhydroxidlösung bei einer Temperatur von 20 bis 100°C, bevorzugt bei 40 bis 85°C, besonders bevorzugt bei 50 bis 80°C. Die dabei anzuwendenden Laugzeiten, d.h. die Reaktionszeiten der Natriumhydroxidlösung mit dem mit Aluminium modifizierten Nickelmetallschaum, können zwischen 2 und 240 Minuten liegen.

In den vorstehend beschriebenen im erfindungsgemäßen Festbett enthaltenen katalytisch aktiven Metallschaumkörper bleibt die makroskopische Schaumstruktur des ursprünglich eingesetzten Metallschaums erhalten. Das mindestens teilweise Herauslösen des Aluminiums aus der Ni/Al Legierung im oben beschriebenen Beispiel erfolgt in Oberflächen-nahen Bereichen, wo eine hochporöse, katalytisch aktive Nickelstruktur erzeugt wird. Die BET-Oberfläche des so erhaltenen katalytisch aktiven Nickelschaumkörpers ist bevorzugt größer als die des eingesetzten Metallschaums vor der Aktivierung.

Ein weiterer Gegenstand der vorliegenden Erfindung ist ein Verfahren zur katalytischen Umsetzung von mindestens einer Eduktkomponente in flüssiger Phase und mindestens einer gasförmigen Komponente in Gegenwart eines erfindungsgemäßen Festbetts enthaltend katalytisch aktive Metallschaumkörper, wobei die katalytisch aktiven Metallschaumkörper ein Volumen von nicht mehr als 500 Millilitern aufweisen und zu mindestens 95 Gew.-% aus Metallen bestehen.

Das vorstehend beschriebene erfindungsgemäße Festbett wird zur katalytischen Umsetzung von mindestens einer Eduktkomponente in flüssiger Phase und mindestens einer gasförmigen Komponente eingesetzt. Solche Dreiphasen-Reaktionen können diskontinuierlich (Batch- oder Satzbetrieb) oder kontinuierlich durchgeführt werden. Bevorzugt wird das erfindungsgemäße Verfahren kontinuierlich durchgeführt.

Alle Reaktortypen, die die räumliche Fixierung des erfindungsgemäßen Festbetts während des Betriebes ermöglichen, können im erfindungsgemäßen Verfahren eingesetzt werden. So können beispielsweise Rührkesselreaktoren, Festbettreaktoren oder andere, dem Fachmann bekannte Apparate benutzt werden.

Wird das erfindungsgemäße Verfahren in einem Rührkesselreaktor durchgeführt, so liegt das erfindungsgemäße Festbett bevorzugt in einer Haltevorrichtung vor. Diese ist vorzugsweise nahe der Rührwelle angeordnet, wobei die Anordnung derart erfolgt, dass durch den Rührer eine Strömung des Reaktionsgemisches durch das in die Haltevorrichtung eingebrachte Festbett erzeugt wird.

Bevorzugt wird das erfindungsgemäße Verfahren kontinuierlich in einem Festbettreaktor durchgeführt, beispielsweise in einem Rieselbett- oder Sumpfreaktor oder in einem anderen, der Fachwelt bekannten Festbettreaktortypen. Alle geeigneten Reaktortypen können in einem "once through"-Modus betrieben werden, wobei die Reaktanden (Feed) in den Reaktor eingeleitet werden, während das Produktgemisch nach im erfindungsgemäßen Festbett erfolgter Reaktion abgeführt wird. Alternativ kann ein Teil des Produktgemisches aus dem Reaktor zurück in die Reaktionszone geleitet werden (Kreislaufstrom). Bei einer solchen Kreislaufführung (Recyclingbetrieb) beträgt das Gewichtsverhältnis von Feed zu Kreislaufstrom 0,025 bis 0,25, bevorzugt 0,05 bis 0,15, besonders bevorzugt 0,05 bis 0,1.

Das erfindungsgemäße Verfahren wird bevorzugt kontinuierlich in einem Reaktor mit Gesamtvolumen von mindestens 0,5 m$^3$ durchgeführt, besonders bevorzugt in einem Reaktor mit einem Gesamtvolumen von 1 bis 500 m$^3$ und ganz besonders bevorzugt 5 bis 100 m$^3$.

Eine katalytische Umsetzung nach dem erfindungsgemäßen Verfahren ist bevorzugt eine Hydrierung mindestens eines Stoffes, der als Eduktkomponente in flüssiger Phase vorliegt und mindestens eine ungesättigte C-C-Bindung und/oder mindestens eine funktionelle Gruppe enthält. Dabei wird Wasserstoff als die gasförmige Komponente eingesetzt.

Die zur Hydrierung eingesetzte Eduktkomponente, die in flüssiger Phase vorliegt, ist dabei vorzugsweise mindestens ein Stoff ausgewählt aus der Gruppe bestehend aus Alkenen, Alkinen, ungesättigten Alkoholen, aromatischen Verbindungen, organischen Nitro- und Nitrosoverbindungen, organischen Isocyanaten, organischen Cyanidverbindungen, Aldehyden und Ketonen. Insbesondere kann es sich bei der katalytischen Umsetzung im erfindungsgemäßen Verfahren um die Hydrierung eines Stoffes aus der Gruppe der Zucker handeln, wobei der zu hydrierende Zucker dann gegebenenfalls in wässriger Lösung dem erfindungsgemäßen Festbett zugeführt wird.

Weiterhin eignet sich das erfindungsgemäße Festbett aus katalytisch aktiven Metallschaumkörpern zur Dehydrierung von Alkoholen zu Aldehyden und/oder Ketonen oder zur reduktive Aminierung von Aldehyden und/oder Ketonen. Auch zur Synthese von Fettaminen oder zur reduktiven Alkylierung organischer Verbindungen kann das erfindungsgemäße Festbett eingesetzt werden.

Weitere Einsatzgebiete des erfindungsgemäßen Festbetts sind heterogen katalysierte Reaktionen in der Gasphase wie die Oxidation von Ethen zu Ethylenoxid oder die Ammoniakoxidation nach dem Prinzip des Ostwaldverfahrens.

Die Erfindung wird im Folgenden anhand einiger Beispiele näher erläutert. Exemplarisch wird die Herstellung und Verwendung einer erfindungsgemäßen Festbettschüttung in der Hydrierung von 1,4-Butindiol zu Butandiol gezeigt.

Beispiel 1

Ein kommerziell als Rollenware erhältlicher Nickelschaum mit einer Dicke von 1,9 mm, einer Breite von 300 mm und einer mittleren Porengröße von 580 μm wurde mit einer kommerziell erhältlichen Haftvermittlerlösung besprüht, mit Aluminiumpulver beschichtet und einer Wärmebehandlung unter Ausschluss von Sauerstoff um 700°C unterzogen. Nach Abkühlung wurde das so erhaltene Material mit einem Laser in viereckige Teilchen mit einer Kantenlänge von 2 mm x 2 mm und einer Dicke von 1,9 mm geschnitten.

Das resultierende Schüttgut wurde zur katalytischen Aktivierung in einer Festbettschüttung angeordnet und durch Durchpumpen von 10 Gew.-%iger Natronlauge nasschemisch bei 80 bis 90°C für die Dauer von 70 Minuten nachbehandelt und anschließend mit Wasser gewaschen, bis ein pH-Wert der Waschlösung nach Durchpumpen durch die Festbettschüttung < 10 erreicht war.

**Vergleichsbeispiel**

Großtechnisch werden in der Hydrierung von 1,4-Butindiol (BYD) zu 1,4-Butandiol aktivierte Nickelkatalysatoren vom Granulattyp eingesetzt, wie beispielsweise in DE 2004611 A offenbart. Ein solcher Katalysator wurde als Vergleichsmaterial hergestellt. Hierzu wurde durch Aufschmelzen von Nickel und Aluminium eine Legierung bestehend aus 50 Gew.-% Nickel und 50 Gew.-% Aluminium hergestellt, mechanisch zerkleinert und ausgesiebt, so dass sich eine Kornfraktion mit einer mittleren Korngröße von 2 mm ergab. Diese Legierungsgranulatfraktion wurde in einer Festbettschüttung durch Durchpumpen von 10 Gew.-%iger Natronlauge bei 60°C für die Dauer von 60 Minuten katalytisch aktiviert und anschließend mit Wasser gewaschen, bis in der resultierenden Waschlösung ein pH-Wert < 10 erreicht war.

Beispiel 2

Die erfindungsgemäßen katalytisch aktiven Metallschaumkörper aus Beispiel 1 und der Katalysator nach dem Stand der Technik aus dem Vergleichsbeispiel wurden in einer kontinuierlichen Festbettanlage auf ihre Hydrieraktivität gegenüber Butin-1,4-diol (BYD) zu 1,4-Butandiol getestet. Dazu wurden sowohl die erfindungsgemäßen katalytisch aktivierten Metallschaumkörper als auch der Granulatkatalysator nach dem Stand der Technik aus dem Vergleichsbeispiel jeweils in einen Rohrreaktor mit einem Innendurchmesser von 12 mm und einer Länge von 175 mm mit einem Effektivvolumen von 18 ml eingefüllt. Das Volumen des eingefüllten Katalysatorfestbetts betrug 10 ml. Der Rohrreaktor war zur Temperierung in einem GC-Ofen angeordnet. Dem Reaktor vorgeschaltet befand sich ein mit Glaskugeln gefülltes Rohr, das als Einlaufstrecke in den Reaktor der Temperierung und Vormischung der Edukte diente.
Bei einem Wasserstoffdruck von 275 bar wurde dem Reaktor kontinuierlich ein Flüssigfeedstrom bestehend aus 50 Gew.-% Wasser, 20 Gew.-% Butin-1,4-diol und 30 Gew.-% 1,4-Butandiol zugeführt, in dem durch Zugabe von verdünnter Natronlauge ein pH-Wert von 7,3 bis 7,5 eingestellt war. Die Raumgeschwindigkeit (Liquid Hourly Space Velocity, LHSV) bei der gewählten Betriebsweise betrug 2,75 h$^{-1}$. Die Temperierung des Reaktors erfolgte auf 120°C.
Während des kontinuierlichen Betriebes des Reaktors über mehrere Tage wurden in regelmäßigen Abständen Proben des Reaktionsproduktes entnommen und per Gaschromatographie auf ihre Zusammensetzung hin untersucht. In Tabelle 1 sind die Ergebnisse dieser kontinuierlichen Tests zusammengefasst:

**Tabelle 1.**

| Versuchslaufzeit in Stunden | Anteil 1,4-Butandiol im Reaktionsprodukt | |
|---|---|---|
| | Katalysator aus Vergleichsbeispiel | Katalysator aus Beispiel 1 |
| 0 | 55,6 % | 55,6 % |
| 4 | 70,5 % | 65,2 % |
| 28 | 75,4 % | 80,7 % |
| 55 | 66,3 % | 93,1 % |

Es wurde festgestellt, dass am erfindungsgemäßen Festbett aus katalytisch aktiven Nickelschaumkörpern höhere katalytische Aktivitäten erreicht werden konnten, als am Festbett nach dem Stand der Technik. Zudem zeigte das erfindungsgemäße Festbetts aus katalytisch aktiven Nickelschaumkörpern deutlich verbesserte Standzeiten, konnte also erheblich länger kontinuierlich auf höherem Aktivitätsniveau betrieben werden, als das Katalysatorfestbett nach dem Stand der Technik.

**Patentansprüche**

1. Ein Festbett enthaltend katalytisch aktive Metallschaumkörper zur katalytischen Umsetzung von mindestens einer Eduktkomponente in flüssiger Phase und mindestens einer gasförmigen Komponente
   **dadurch gekennzeichnet, dass**
   die katalytisch aktiven Metallschaumkörper ein Volumen von nicht mehr als 500 Millilitern aufweisen und zu mindestens 95 Gew.-% aus Metallen bestehen.

2. Festbett nach Anspruch 1,

**dadurch gekennzeichnet, dass**
die katalytisch aktiven Metallschaumkörper ein oder mehrere Metalle ausgewählt aus der Gruppe bestehend aus Nickel, Cobalt, Eisen, Silber, Platin, Chrom, Molybdän und Wolfram enthalten.

3. Festbett nach Anspruch 2,
**dadurch gekennzeichnet, dass**
die katalytisch aktiven Metallschaumkörper zusätzlich bis zu 25 Gew.-% Al enthalten.

4. Festbett nach einem der Ansprüche 1 bis 3,
**dadurch gekennzeichnet, dass**
die katalytisch aktiven Metallschaumkörper einen Sauerstoffgehalt von nicht mehr als 7500 ppm aufweisen.

5. Festbett nach einem der Ansprüche 1 bis 4,
**dadurch gekennzeichnet, dass**
die katalytisch aktiven Metallschaumkörper in dem Festbett als Schüttgut vorliegen, wobei ein Volumen von 1 L dieses Schüttgutes nicht mehr als 0,8 kg Gewicht aufweist.

6. Festbett nach einem der Ansprüche 1 bis 5,
**dadurch gekennzeichnet, dass**
die katalytisch aktiven Metallschaumkörper als zylindrische, ringförmige, quader-, paralleliped- oder würfelförmige Körper vorliegen.

7. Festbett nach Anspruch 6,
**dadurch gekennzeichnet, dass**
die katalytisch aktiven Metallschaumkörper zylindrische Form aufweisen, wobei die zylindrische Form durch Wicklung einer Metallschaumplatte erzeugt worden ist.

8. Festbett nach Anspruch 7,
**dadurch gekennzeichnet, dass**
die Metallschaumplatte vor Wicklung in eine gewellte Form gebracht worden ist.

9. Festbett nach einem der Ansprüche 1 bis 8,
**dadurch gekennzeichnet, dass**
die katalytisch aktiven Metallschaumkörper makroskopische Poren mit einer Größe im Bereich von 100 bis 5000 $\mu$m aufweisen.

10. Festbett nach einem der Ansprüche 1 bis 9,
**dadurch gekennzeichnet, dass**
die katalytisch aktiven Metallschaumkörper eine BET-Oberfläche von 1 bis 200 $m^2$/g aufweisen.

11. Verfahren zur katalytischen Umsetzung von mindestens einer Eduktkomponente in flüssiger Phase und mindestens einer gasförmigen Komponente in Gegenwart des Festbetts gemäß einem der Ansprüche 1 bis 10.

12. Verfahren nach Anspruch 11,
**dadurch gekennzeichnet, dass**
das Verfahren kontinuierlich in einem Reaktor mit Gesamtvolumen von mindestens 0,5 $m^3$ durchgeführt wird.

13. Verfahren nach einem der Ansprüche 11 oder 12,
**dadurch gekennzeichnet, dass**
die katalytische Umsetzung eine Hydrierung ist, wobei Wasserstoff die gasförmige Komponente ist, und die Eduktkomponente in flüssiger Phase mindestens eine ungesättigte C-C-Bindung und/oder mindestens eine funktionelle Gruppe enthält.

14. Verfahren nach einem der Ansprüche 11 bis 13,
**dadurch gekennzeichnet, dass**
die katalytische Umsetzung eine Hydrierung mindestens eines Stoffes ausgewählt aus der Gruppe bestehend aus Alkenen, Alkinen, ungesättigten Alkoholen, aromatischen Verbindungen, organischen Nitro- und Nitrosoverbindungen, organischen Isocyanaten, organischen Cyanidverbindungen, Aldehyden und Ketonen, ist, und wobei Wasserstoff als gasförmige Komponente eingesetzt wird.

15. Verfahren nach Anspruch 14,
**dadurch gekennzeichnet, dass**
die katalytische Umsetzung die Hydrierung eines Stoffes aus der Gruppe der Zucker ist.

16. Verfahren nach einem der Ansprüche 11 oder 12,
**dadurch gekennzeichnet, dass**
die katalytische Umsetzung eine Dehydrierung von Alkoholen zu Aldehyden und/oder Ketonen oder eine reduktive Aminierung von Aldehyden und/oder Ketonen ist.

17. Verfahren nach einem der Ansprüche 11 oder 12,
**dadurch gekennzeichnet, dass**
die katalytische Umsetzung eine Synthese von Fettaminen oder eine reduktive Alkylierung organischer Verbindungen ist.

Europäisches
Patentamt

European
Patent Office

Office européen
des brevets

# EUROPÄISCHER RECHERCHENBERICHT

**Nummer der Anmeldung**

EP 16 19 1751

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | LIU YA-ZHAO ET AL: "Monolithic catalysts with Pd deposited on a structured nickel foam packing", CATALYSIS TODAY, ELSEVIER, AMSTERDAM, NL, Bd. 273, 7. April 2016 (2016-04-07), Seiten 34-40, XP029580146, ISSN: 0920-5861, DOI: 10.1016/J.CATTOD.2016.03.017 * Zusammenfassung * * Abs. 2. "Experimental" * * Tabelle 1 *  ----- | 1-17 | INV. B01J23/755 B01J25/02 B01J35/00 B01J35/02 B01J35/04 B01J35/10 B01J19/00 B01J23/16 B01J23/70 C07C31/02 C07C1/20 |
| X | COLEMAN L J I ET AL: "Evaluation of Foam Nickel for the Catalytic Partial Oxidation of Methane", CATALYSIS LETTERS, KLUWER ACADEMIC PUBLISHERS-PLENUM PUBLISHERS, NE, Bd. 128, Nr. 1-2, 26. November 2008 (2008-11-26), Seiten 144-153, XP019671963, ISSN: 1572-879X * par. 2. "Experimental" * * Abbildung 1 *  ----- | 1-10 | |
| X | WO 2005/039764 A1 (DEGUSSA [DE]; OSTGARD DANIEL [DE]; BERWEILER MONIKA [DE]; ROEDER STEFA) 6. Mai 2005 (2005-05-06) * Beispiele * * Seite 13, Zeile 31 - Seite 14, Zeile 24 * * Seite 16, Zeile 3 - Seite 17, Zeile 25 * * Tabellen 4,6 *  -----  -/-- | 1-17 | RECHERCHIERTE SACHGEBIETE (IPC) B01J C07C |

Der vorliegende Recherchenbericht wurde für alle Patentansprüche erstellt

| Recherchenort | Abschlußdatum der Recherche | Prüfer |
|---|---|---|
| München | 14. März 2017 | Omegna, Anna |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

................................................................

& : Mitglied der gleichen Patentfamilie, übereinstimmendes Dokument

EPO FORM 1503 03.82 (P04C03)

Europäisches
Patentamt

European
Patent Office

Office européen
des brevets

**EUROPÄISCHER RECHERCHENBERICHT**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | WO 2008/151614 A2 (EADS DEUTSCHLAND GMBH [DE]; BOETTIGER MICHAEL [CH]; JEHLE WALTER [DE]) 18. Dezember 2008 (2008-12-18) <br> * Zusammenfassung * <br> * Seite 1, Zeile 9 - Zeile 13 * <br> * Seite 6, Zeile 18 - Zeile 20 * <br> * Beispiele * <br> * Ansprüche * <br> ----- | 1-17 | |

RECHERCHIERTE SACHGEBIETE (IPC)

Der vorliegende Recherchenbericht wurde für alle Patentansprüche erstellt

| Recherchenort | Abschlußdatum der Recherche | Prüfer |
|---|---|---|
| München | 14. März 2017 | Omegna, Anna |

KATEGORIE DER GENANNTEN DOKUMENTE

X : von besonderer Bedeutung allein betrachtet
Y : von besonderer Bedeutung in Verbindung mit einer
  anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
  nach dem Anmeldedatum veröffentlicht worden ist
D : in der Anmeldung angeführtes Dokument
L : aus anderen Gründen angeführtes Dokument
.............................................................................
& : Mitglied der gleichen Patentfamilie, übereinstimmendes
  Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 16 19 1751

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

14-03-2017

| Im Recherchenbericht angeführtes Patentdokument | Datum der Veröffentlichung | Mitglied(er) der Patentfamilie | Datum der Veröffentlichung |
|---|---|---|---|
| WO 2005039764 A1 | 06-05-2005 | AU 2003276141 A1<br>WO 2005039764 A1<br>WO 2005042153 A1 | 19-05-2005<br>06-05-2005<br>12-05-2005 |
| WO 2008151614 A2 | 18-12-2008 | DE 102007027837 A1<br>WO 2008151614 A2 | 18-12-2008<br>18-12-2008 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 10245510 A1
- DE 2004611 A

**In der Beschreibung aufgeführte Nicht-Patentliteratur**

- Heterogeneous Catalysis and Solid Catalysts, 3. Industrial Applications. Ullmann's Encyclopedia of Industrial Chemistry. 15. Oktober 2011
- Catalytic Fixed-Bed Reactors. Ullmann's Encyclopedia of Industrial Chemistry. 15. Juli 2012
- Heterogeneous Catalysis and Solid Catalysts, 2. Development and Types of Solid Catalysts. Ullmann's Encyclopedia of Industrial Chemistry. 15. Oktober 2011
- Metallic Foams. Ullmann's Encyclopedia of Industrial Chemistry. 15. Juli 2012
- *AICHE Journal,* 2015, vol. 61 (12), 4323-4331
- *In Catalysis Today,* 2016, vol. 273, 221-233
- *ACS Catal.,* 2016, vol. 6, 5432-5440
- The Guide 2000 of Technical Foams. 33-41
- *J. Am. Chem. Soc.,* 1938, vol. 60, 309-319